# EUROPEAN PATENT SPECIFICATION

(11) **EP 2 682 175 B1**
(45) Date of publication and mention of the grant of the patent: **29.04.2015**
(21) Application number: 13174420.3
(22) Date of filing: 28.06.2013
(51) Int. Cl.: B01D 53/66, B01D 53/86, A61L 2/20, B01D 53/34

(54) **Plant and method for oxidization using ozone and abatement thereof**
Anlage und Verfahren zur Oxidation unter Verwendung von Ozon und Minderung davon
Installation et procédé d'oxydation utilisant de l'ozone et la réduction duquel

(30) Priority: 06.07.2012 IT UD20120123
(43) Date of publication of application: 08.01.2014
(73) Proprietor: Martik SRL, 33030 Coseano (UD) (IT)
(72) Inventor: Di Bidino, Virginio, 33030 Rive d'Arcano (UD) (IT)
(74) Representative: Petraz, Gilberto Luigi

(56) References cited:
- EP-A2- 1 650 500
- DE-A1- 4 024 271
- JP-A- 8 151 202
- JP-A- 9 315 804
- JP-A- 59 042 025

## Description

### FIELD OF THE INVENTION

The present invention concerns a plant and method for oxidization using ozone and the abatement thereof, used in particular to decontaminate objects contained in a suitable treatment cell.

### BACKGROUND OF THE INVENTION

Ozone (symbol O₃) is an allotropic form of oxygen, with a triatomic molecule and molecular weight of 48. Ozone occurs in normal conditions as a blue gas, with an acrid odor, and has a strong oxidizing power. Ozone is prepared with ozonizers, which for example operate with known electric discharges as a crown effect, or by subjecting the air or oxygen to high-frequency ultraviolet irradiation. It is used as a disinfectant, deodorant, bactericide and sterilizer, especially of water, or as an oxidant in numerous organic syntheses.

It is known to use a stream of ozone as an oxidant and decontamination agent for objects or products contained in a treatment cell. Due to its chemical properties, ozone can be irritating or toxic, if in an excessive concentration, for the human body or animals.

It is therefore necessary, downstream of the treatment chambers, to abate the ozone content, so that the spent stream can be discharged into the environment without causing damage or pollution. Generally, it is provided to use active carbon filters, but these are expensive, since they need maintenance to be regenerated and in any case they have to be replaced, after a certain working life, with the problem of having to dispose of the spent material.

At the same time, it is in any case necessary to make the decontamination process using ozone effective and reliable.

Document JP-A-59042025 describes a known apparatus for removing ozone from a gas.

One purpose of the present invention is to obtain an oxidization plant using ozone and the abatement thereof, and the corresponding method, used in particular for the decontamination using oxidization of objects contained in a suitable treatment cell, which allows to discharge the spent stream with a reduced, if not zero ozone content, so that it is not irritating or toxic and does not cause damage to persons or pollute the environment.

Another purpose of the present invention is to obtain a plant for oxidization using ozone and the abatement thereof, and the corresponding method, that is effective in the decontamination treatment using oxidization and that reduces the energy costs connected to its functioning.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a plant for oxidization using ozone and the abatement thereof according to the present invention is used for the decontamination using oxidization of objects contained in a treatment cell.

The plant according to the present invention comprises not only the treatment cell but also an ozone generator fed at inlet by a stream of air.

Moreover, the plant comprises a cell to abate the ozone disposed downstream of the treatment cell to receive the stream of ozone that passes into the treatment cell by means of oxidization before it is discharged into the environment.

Moreover, the plant comprises a heat pump, to transfer heat energy exploiting changes in the thermodynamic state of a heat-carrying fluid. The heat pump cooperates on one side with the treatment cell and on the other side with the abatement cell. The heat pump is configured to determine a cooling inside the treatment cell, advantageous for the purposes of stabilizing the oxidization using ozone and thus to allow increased effectiveness thereof in the decontamination action during treatment.

The heat pump is also configured to determine, in the abatement cell, a heating that is effective for abating the ozone content in the stream of ozone transiting in the abatement cell and thus to obtain an abatement, or at least a reduction if not substantial elimination, of the ozone content in the stream exiting from the abatement cell.

In one form of embodiment, the heat pump is configured to cool the inside of the treatment cell to a temperature lower than about 12°C, advantageous for the purposes of the decontamination action performed by the ozone, and to heat the inside of the abatement cell to a temperature higher than at least about 58°C, advantageous for the purposes of abating the ozone.

A method for oxidization using ozone and the abatement thereof, used for the decontamination using oxidization of objects contained in an oxidization treatment cell, comprises a generation of ozone and a treatment with the ozone generated of the objects in the treatment cell.

The method comprises the abatement of the ozone downstream of the treatment, before the ozone is discharged into the environment or atmosphere. The method provides to use a heat pump that is selectively activated to cool the inside of the oxidization treatment cell during the treatment of the objects using ozone, and to heat the stream of ozone arriving from the treatment cell during the abatement of the ozone, so as to reduce if not eliminate the ozone content therein and thus to obtain an abatement of the ozone content in the exiting stream.

### DESCRIPTION OF SOME FORMS OF EMBODIMENT

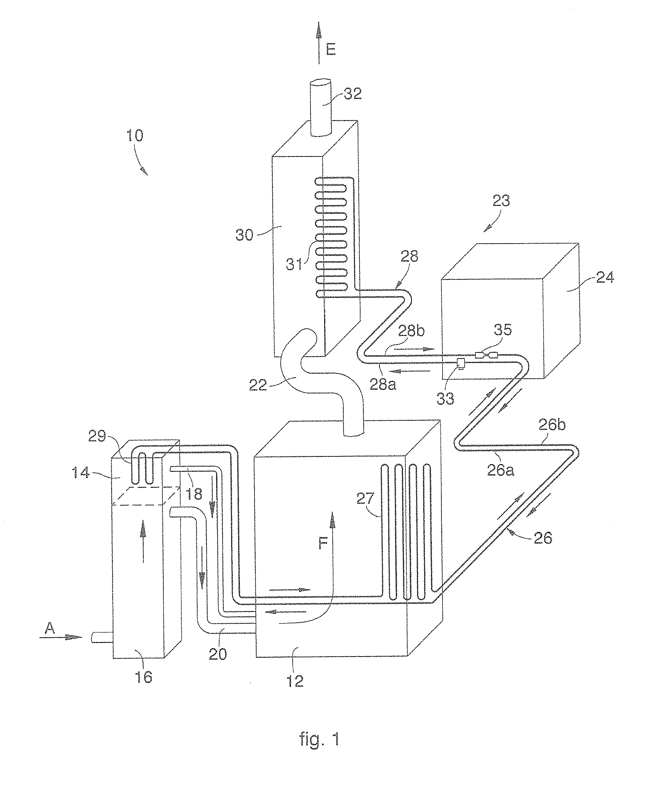

We shall now refer in detail to the various forms of embodiment of the present invention, of which one or more examples are shown in the attached drawing. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

These and other characteristics of the present invention will become apparent from the following description of some forms of embodiment, given as a non-restrictive example with reference to the attached drawing which shows a plant 10 for oxidization using ozone and abatement thereof, used in particular for the decontamination of objects contained in a suitable treatment cell 12.

The treatment cell 12 is preferably made as the sealed type, in any case watertight, and provides suitable closable apertures, for the insertion of the objects to be treated and may provide means to extract and discharge the stream of ozone used for the treatment.

The objects that can be treated in the treatment cell 12 according to the present invention are of any type that need disinfection by means of oxidization or decontamination in order to be used or re-used. These include, by way of non-restrictive example, analysis instruments, laboratory, surgical or other instruments, and also food products, pharmaceuticals, cosmetics or suchlike or comparable, as well as devices, equipment, metal articles, machines or components used for the production or working thereof, but also personal care products or clothing, including professional clothing or other. It goes without saying that the present invention, which inevitably causes a decontamination action using oxidization also on the internal walls of the treatment cell 12, can also be used to decontaminate the inside of rooms, containers or similar limited spaces which, suitably adapted, possibly sealed, represent in themselves the treatment cell 12.

The plant 10 according to the present invention comprises the treatment cell 12 and also comprises, an ozone generator 14 fed at inlet by a stream of air, as indicated by arrow A.

Upstream of the ozone generator 14 a filter 16 may be provided, for example the HEPA type, to filter the air introduced before it enters into the ozone generator 14.

The ozone generator 14 sends the ozone produced at entry to the oxidization treatment cell 12 by means of a first ozone delivery pipe 18. The stream F of ozone in the treatment cell 12 performs its decontaminating action by means of oxidization at least on the objects contained in the treatment cell 12.

Moreover, a second delivery pipe 20, in this case exiting from the filter 16, is provided to introduce a stream of washing air, inside the treatment cell 12.

The treatment cell 12 is also provided with an outlet or discharge pipe 22 for the fluid contained therein, after the oxidization treatment has been carried out. The movement of the stream of ozone and/or air along the plant can be guaranteed by suitable aeraulic machines, not shown in the drawings.

The plant 10 also comprises an ozone abatement cell 30, disposed downstream of the oxidization treatment cell 12 and connected to the exit of the latter by means of the outlet pipe 22. The abatement cell 30 also has, downstream, a final discharge 32 from which the stream, with its limited if not zero ozone content, exits as indicated by the arrow E.

The plant 10 comprises a heat pump 23, by means of which both to cool the ozone entering the treatment cell 12 and also the treatment cell 12 itself, so that the ozone, at lower temperatures, is more effective in its decontamination action; and also to heat the abatement cell 30 and hence the stream of ozone transiting therein, so as to reduce, if not eliminate, the ozone content therein.

Indeed, since ozone is a molecule that gradually becomes more unstable as the temperature increases, it is advantageous on the one hand to cool the stream of ozone and the oxidization treatment cell 12 in which it operates, in order to ensure the stability thereof and hence its effectiveness during decontamination, and on the other hand to render the ozone deliberately unstable, raising its temperature in the abatement cell 30 once it has come out of the treatment cell 12, and before being discharged into the environment, so as to abate the content thereof.

Furthermore, achieving the targeted cooling and heating using the heat pump 23 is very advantageous from the energy point of view, allowing a considerable economic saving.

In this way it is possible to ensure the effectiveness of the ozone treatment, containing the energy costs and guaranteeing an emission of the spent stream that does not damage persons, animals or the environment.

In particular, for the purposes of effectiveness of the decontamination action of the ozone, it is advantageous to cool the inside of the treatment cell 12 to a temperature lower than about 12°C, preferably lower than about 10°C. For example, a possible functioning range of cooling is between about 5°C and about 10°C.

On the contrary, for the purposes of effectiveness of the abatement action on the ozone, it is advantageous to heat the inside of the abatement cell 30 to a temperature higher than about 58°C, preferably higher than about 60°C, even more preferably higher than about 70°C, most preferably higher than 90°C. For example, a possible heating functioning range is between about 65°C and about 105°C.

The heat pump 23 is suitably designed and sized to obtain the desired cooling and heating.

In one form of embodiment, the heat pump 23 comprises a compression and expansion unit 24, in which a compressor 33 and an expansion valve 35 are provided, to determine the necessary steps of expansion and compression on a heat-carrying fluid in circulation, by means of which to obtain the desired cooling and heating.

The compression and expansion unit 24 is connected on one side to a first cold circuit 26, in which the heat-carrying fluid circulates and is directed toward the oxidization treatment cell 12, and a second hot circuit 28, in which the heat-carrying fluid circulates and is directed toward the abatement cell 30.

The first cold circuit 26 extends inside the oxidization treatment cell 12, where it is associated with a first evaporator 27, made as a heat exchanger suitably designed and sized to obtain the desired cooling, in particular to reach a temperature lower than at least about 15°C as described above.

The first cold circuit 26 comprises two cold branches 26a, 26b, of which a first cold branch 26a between the first evaporator 27 and the compressor 33, and a second cold branch 26b between the expansion valve 35 and the first evaporator 27.

Furthermore, in the form of embodiment shown by way of example, the first cold circuit 26 also develops inside the ozone generator 14, where it is associated with a second evaporator 29, which in this case can be analogous to the first evaporator 27 but smaller in size. In particular, an initial segment of the first cold branch 26a is provided between the second evaporator 29 and the first evaporator 27, while a subsequent segment of the first cold branch 26a is between the first evaporator 27 and the compressor 33. In this case, the second cold branch 26b is connected to the second evaporator 29, that is, it develops between the expansion valve 35 and the second evaporator 29.

The second hot circuit 28 extends inside the abatement cell 30, where it is associated with a capacitor 31, also made as a heat exchanger suitably designed and sized to obtain the desired heating, in particular to reach a temperature higher than at least about 58°C as described above.

The second hot circuit 28 comprises two hot branches 28a, 28b, of which a first hot branch 28a between the compressor 33 and the capacitor 31 and a second hot branch 28b between the capacitor 31 and the expansion valve 35.

The compressor 33, through the first cold branch 26a of the first cold circuit 26, takes the heat-carrying fluid from the first evaporator 27 and possibly also from the second evaporator 29 if provided, where the fluid evaporates at low pressure, absorbing heat and thus cooling the inside of the treatment cell 12 so as to render the decontamination oxidization action of the ozone more effective. This determines the cooling in the treatment cell 12 and possibly in the ozone generator 14.

Subsequently, the compressor 33 thrusts the heat-carrying fluid through the first hot branch 28a of the second hot circuit 28, into the capacitor 31 where the fluid condenses, at high pressure, releasing the heat absorbed and heating the inside of the abatement cell 30 so as to abate the ozone content in the exiting stream.0

The function of the expansion valve 35, which receives the heat-carrying fluid exiting from the capacitor 31 so as to send it to the first evaporator 27 and possibly also to the second evaporator 29 if provided, is to allow the expansion of the fluid for the subsequent evaporation at low pressure.

The heat-carrying fluid therefore changes its thermodynamic state inside the two heat exchangers, passing in the first evaporator 27 and possibly also in the second evaporator 29 if provided, from liquid to gas and in the capacitor 31 from gas to liquid.

## Claims

1. Plant for oxidization using ozone and the abatement thereof comprising an oxidization treatment cell (12), which can be used to decontaminate by means of oxidization objects contained in said oxidization treatment cell (12), and comprising an ozone generator (14) fed in input by a stream of air (A), **characterized in that** it comprises an ozone abatement cell (30) disposed downstream of the oxidization treatment cell (12) in order to receive the stream of ozone (F) which passes into the oxidization treatment cell (12) before being discharged into the environment, and a heat pump (23) which cooperates on one side with the oxidization treatment cell (12) and on the other side cooperates with the abatement cell (30) and configured to determine a cooling inside the oxidization treatment cell (12) and, in the abatement cell (30), an effective heating to abate the ozone content in the stream of ozone (F) which transits in the abatement cell (30) and to therefore obtain an abatement of the ozone content in the stream (E) exiting from the abatement cell (30).

2. Plant as in claim 1, **characterized in that** the heat pump (23) is configured to cool the inside of the oxidization treatment cell (12) to a temperature lower than about 12°C and to heat the inside of the abatement cell (30) to a temperature higher than at least about 58°C.

3. Plant as in claim 1 or 2, **characterized in that** it comprises a first ozone delivery pipe (18) from the ozone generator (14) into the oxidization treatment cell (12).

4. Plant as in claim 3, **characterized in that** it also comprises a second delivery pipe (20) to introduce a stream of air inside the oxidization treatment cell (12).

5. Plant as in claim 4, **characterized in that** the oxidization treatment cell (12) is also provided with an outlet pipe (22) connected to the abatement cell (30).

6. Plant as in any claim hereinbefore, **characterized in that** the heat pump (23) comprises a compression and expansion unit (24), in which a compressor (33) and an expansion valve (35) are provided.

7. Plant as in claim 6, **characterized in that** the compression and expansion unit (24) is connected on one side to a first cold circuit (26), in which the heat carrying fluid that is directed toward the oxidization treatment cell (12) circulates, and a second hot circuit (28), in which the heat carrying fluid that is directed toward the abatement cell (30) circulates.

8. Plant as in claim 7, **characterized in that** the heat pump (23) comprises at least a first evaporator (27) associated to the oxidization treatment cell (12) and the first cold circuit (26) comprises a first cold branch (26a) at least between the at least one evaporator (27) and the compressor (33), and a second cold branch (26b) between the expansion valve (35) and the first evaporator (27).

9. Plant as in claim 7, **characterized in that** the heat pump (23) comprises at least a first evaporator (27) associated with the oxidization treatment cell (12) and a second evaporator (29) associated inside the ozone generator (14), and wherein the first cold circuit (26) comprises a first cold branch (26a) in which an initial segment of the first cold branch (26a) is provided between the second evaporator (29) and the first evaporator (27), while a subsequent segment of the first cold branch (26a) is between the first evaporator (27) and the compressor (33), and a second cold branch (26b) is between the expansion valve (35) and the second evaporator (29).

10. Plant as in claim 7, 8 or 9, **characterized in that** the heat pump (23) comprises at least a capacitor (31) and the second hot circuit (28) comprises a first hot branch (28a) between the compressor (33) and the capacitor (31) and a second hot branch (28b) between the capacitor (31) and the expansion valve (35).

11. Oxidization method using ozone and the abatement thereof, which can be used to decontaminate by means of oxidization objects contained in an oxidization treatment cell (12), comprising the generation of ozone and an oxidization treatment, with the ozone generated, of the objects in the oxidization treatment cell (12), **characterized in that** it comprises an abatement of the ozone downstream of the oxidization treatment, before the ozone is discharged into the environment, said method providing to use a heat pump (23) which is activated to cool the inside of the oxidization treatment cell (12) during the ozone treatment of the objects and to heat the stream of ozone (F) arriving from the oxidization treatment cell (12) during the ozone abatement, in order to reduce the ozone content and thus to obtain an abatement of the ozone content in the outlet stream (E).

## Patentansprüche

1. Anlage zur Oxidation mittels Ozon und dessen Verringerung, welche aufweist
eine Oxidationsbehandlungszelle (12), welche zum Dekontaminieren von in der Oxidationsbehandlungszelle (12) enthaltenen Oxidationsobjekten verwendet werden kann,
und einen Ozonerzeuger (14), welcher am Eingang mit einem Luftstrom (A) gespeist wird,
**dadurch gekennzeichnet, dass** sie aufweist
eine Ozonverringerungszelle (30), welche stromabwärts der Oxidationsbehandlungszelle (12) angeordnet ist, zum Aufnehmen des Ozonstroms (F), welcher in die Oxidationsbehandlungszelle (12) strömt, bevor dieser in die Umwelt entlassen wird,
und eine auf einer Seite mit der Oxidationsbehandlungszelle (12) kooperierende und auf der anderen Seite mit der Verringerungszelle (30) kooperierende Wärmepumpe (23),
welche konfiguriert ist, ein Kühlen im Inneren der Oxidationsbehandlungszelle (12), und in der Verringerungszelle (30), ein effektives Heizen zum Verringern des Ozongehalts in dem Ozonstrom (F), welcher die Verringerungszelle (30) durchquert, festzulegen,
und somit eine Verringerung des Ozongehalts in dem Strom (E), welcher aus der Verringerungszelle (30) austritt, zu erhalten.

2. Anlage gemäß Anspruch 1, **dadurch gekennzeichnet, dass** die Wärmepumpe (23) konfiguriert ist, das Innere der Oxidationsbehandlungszelle (12) auf eine Temperatur unterhalb von etwa 12° C zu kühlen
und das Innere der Verringerungszelle (30) auf eine Temperatur oberhalb von zumindest etwa 58° C aufzuheizen.

3. Anlage gemäß Anspruch 1 oder 2, **dadurch gekennzeichnet, dass** sie eine erste Ozonförderleitung (18) von dem Ozonerzeuger (14) in die Oxidationsbehandlungszelle (12) aufweist.

4. Anlage gemäß Anspruch 3, **dadurch gekennzeichnet, dass** sie ferner eine zweite Ozonförderleitung (20) zum Einleiten eines Luftstroms in die Oxidationsbehandlungszelle (12) aufweist.

5. Anlage gemäß Anspruch 4, **dadurch gekennzeichnet, dass** die Oxidationsbehandlungszelle (12) ferner mit einer Auslassleitung (22) versehen ist, welche mit der Verringerungszelle (30) verbunden ist.

6. Anlage gemäß einem der vorstehenden Ansprüche, **dadurch gekennzeichnet, dass** die Wärmepumpe (23) eine Kompressions- und Expansionseinheit (24) aufweist, in welcher ein Kompressor (33) und ein Expansionsventil (35) bereitgestellt sind.

7. Anlage gemäß Anspruch 6, **dadurch gekennzeichnet, dass** die Kompressions- und Expansionseinheit (24) auf einer Seite mit
einem ersten Kühlkreislauf (26) verbunden ist, in welchem das wärmetragende Fluid, das in Richtung der Oxidationsbehandlungszelle (12) geleitet wird, zirkuliert,
und einem zweiten Heizkreislauf (28) verbunden ist, in welchem das wärmetragende Fluid, das in Richtung der Verringerungszelle (30) geleitet wird, zirkuliert.

8. Anlage gemäß Anspruch 7, **dadurch gekennzeichnet, dass** die Wärmepumpe (23) zumindest einen ersten Verdampfer (27) aufweist, welcher der Oxidationsbehandlungszelle (12) zugeordnet ist
und der erste Kühlkreislauf (26) ein ersten Kühlrohrabzweig (26a) zumindest zwischen dem zumindest einen Verdampfer (27) und dem Kompressor (33),
und einen zweiten Kühlrohrabzweig (26b) zwischen dem Expansionsventil (35) und dem ersten Verdampfer (27) aufweist.

9. Anlage gemäß Anspruch 7, **dadurch gekennzeichnet, dass** die Wärmepumpe (23) zumindest einen ersten Verdampfer (27) aufweist, welcher der Oxidationsbehandlungszelle (12) zugeordnet ist
und einen zweiten Verdampfer (29) aufweist, welcher im Inneren des Ozonerzeugers (14) angeordnet ist,
und wobei der erste Kühlkreislauf (26) einen ersten Kühlrohrabzweig (26a) aufweist, in welchem ein Initialsegment des ersten Kühlrohrabzweigs (26a) zwischen dem zweiten Verdampfer (29) und dem ersten Verdampfer (27) angeordnet ist, während ein nachfolgendes Segment des ersten Kühlrohrabzweigs (26a) zwischen dem ersten Verdampfer (27) und dem Kompressor (33) ist, und ein zweiter Kühlrohrabzweig (26b) zwischen dem Expansionsventil (35) und dem zweiten Verdampfer (29) ist.

10. Anlage gemäß Anspruch 7, 8 oder 9, **dadurch gekennzeichnet, dass** die Wärmepumpe (23) zumindest einen Kondensator (31) aufweist
und der zweite Heizkreislauf (28) einen ersten Heizrohrabzweig (28a) zwischen dem Kompressor (33) und dem Kondensator (31),
und einen zweiten Heizrohrabzweig (28b) zwischen dem Kondensator (31) und dem Expansionsventil (35) aufweist.

11. Oxidationsverfahren mittels Ozon und dessen Verringerung, welches zum Dekontaminieren von in einer Oxidationsbehandlungszelle (12) enthaltenen Oxidationsobjekten verwendet werden kann, welches die Ozonerzeugung und eine Ozonbehandlung der Objekte mit dem erzeugten Ozon in der Oxidationsbehandlungszelle (12) aufweist,
**dadurch gekennzeichnet, dass**
es eine Verringerung des Ozons stromabwärts der Oxidationsbehandlung aufweist, bevor das Ozon in die Umwelt entlassen wird,
wobei das Verfahren das Verwenden einer Wärmepumpe (23) bereitstellt, welche zum Kühlen des Inneren der Oxidationsbehandlungszelle (12) während der Ozonbehandlung der Objekte
und zum Heizen des Ozonstroms (F), welcher von der Oxidationsbehandlungszelle (12) eintrifft, während der Ozonverringerung
verwendet wird,
um dem Ozongehalt zu reduzieren und somit eine Verringerung des Ozongehalts in dem Auslassstrom (E) zu erhalten.

## Revendications

1. Installation d'oxydation à l'ozone et d'abattement de celui-ci, comprenant une cellule de traitement par oxydation (12), qui peut être utilisée pour décontaminer par oxydation des objets contenus dans ladite cellule de traitement par oxydation (12), et comprenant un générateur d'ozone (14) recevant un flux d'air (A), **caractérisé en ce qu**'elle comprend un cellule d'abattement d'ozone (30), agencée en aval de la cellule de traitement par oxydation (12), pour recevoir le flux d'ozone (F) qui passe par la cellule de traitement par oxydation (12) avant d'être déchargée dans l'environnement, et une pompe à chaleur (23) coopérant d'un côté avec la cellule de traitement par oxydation (12) et de l'autre côté avec la cellule d'abattement (30) et configurée pour déterminer un refroidissement à l'intérieur de la cellule de traitement par oxydation (12) et, dans la cellule d'abattement (30), un réchauffement efficace pour abattre la teneur en ozone dans le flux d'ozone (F) transitant par la cellule d'abattement (30) et pour obtenir ainsi un abattement de la teneur en ozone dans le flux (E) qui sort de la cellule d'abattement.

2. Installation selon la revendication 1, **caractérisé en ce que** la pompe à chaleur (23) est configurée pour refroidir l'intérieur de la cellule de traitement par oxydation (12) à une température au-dessous de 12°C environ et à réchauffer l'intérieur de la cellule d'abattement (30) à une température au-dessus de 58°C environ.

3. Installation selon la revendication 1 ou 2, **caractérisé en ce qu**'elle comprend un premier tuyau d'alimentation d'ozone (18) du générateur d'ozone (14) à la cellule de traitement par oxydation (12).

4. Installation selon la revendication 3, **caractérisé en ce qu**'elle comprend par ailleurs un second tuyau d'alimentation (20) pour introduire un flux d'air à l'intérieur de la cellule de traitement par oxydation (12).

5. Installation selon la revendication 4, **caractérisé en ce que** la cellule de traitement par oxydation (12) est munie aussi d'un tuyau d'émission (22) relié à la cellule d'abattement (30).

6. Installation selon l'importe laquelle des revendications précédentes, **caractérisé en ce que** la pompe à chaleur comprend une unité de compression/d'expansion (24), dans laquelle sont prévus un compresseur (33) et une soupape d'expansion (35).

7. Installation selon la revendication 6, **caractérisé en ce que** l'unité de compression/d'expansion (24) est reliée d'un côté à un premier circuit froid (26), dans lequel circule le fluide caloporteur destiné à la cellule de traitement par oxydation (12) et un second circuit chaud (28), dans lequel circule le fluide caloporteur destiné à la cellule d'abattement (12).

8. Installation selon la revendication 7, **caractérisé en ce que** la pompe à chaleur (23) comprend au moins un premier évaporateur (27) associé à la cellule de traitement par oxydation (12) et le premier circuit froid (26) comprend une première branche froide (26a) au moins entre l'au moins un évaporateur (27) et le compresseur (33), et une seconde branche froide (26b) entre la soupape d'expansion (35) et le premier évaporateur (27).

9. Installation selon la revendication 7, **caractérisé en ce que** la pompe à chaleur (23) comprend au moins un premier évaporateur (27) associé à la cellule de traitement par oxydation (12) et un second évaporateur (29) associé à l'intérieur du générateur d'ozone (14), et dans laquelle le premier circuit froid (26) comprend une première branche froide (26a) dans la quelle un segment initial de la première branche froide (26a) est prévue entre le second évaporateur (29) et le premier évaporateur (27), tandis qu'un segment suivant de la première branche froide (26a) est situé entre le premier évaporateur (27) et le compresseur (33), et une seconde branche froide (26b) est située entre la soupape d'expansion (35) et le premier évaporateur (27).

10. Installation selon la revendication 7, 8 ou 9, **caractérisé en ce que** la pompe à chaleur (23) comprend une première branche chaude (28a) entre le compresseur (33) et le condensateur (31) et une seconde branche chaude (28b) entre le condensateur (31) et la soupape d'expansion (35).

11. Procédé d'oxydation par ozone et d'abattement d'ozone, qui peut être utilisé pour décontaminer par oxydation des objets contenus dans une cellule de traitement par oxydation (12), comprenant la génération d'ozone et un traitement par oxydation, au moyen de l'ozone généré, des objets à l'intérieur de la cellule de traitement par oxydation (12), **caractérisé en ce qu**'il comprend un abattement d'ozone en aval du traitement par oxydation avant la décharge de l'ozone dans l'environnement, ledit procédé comportant l'emploi d'une pompe à chaleur (23), actionnée pour refroidir l'intérieur de la cellule de traitement par oxydation (12) pendant le traitement par ozone des objets, et pour réchauffer un flux d'ozone (F) provenant de la cellule de traitement par oxydation (12) pendant l'abattement d'ozone, afin de réduire la teneur en ozone et d'obtenir ainsi un abattement de la teneur en ozone dans le flux en sortie (E).
